# EUROPÄISCHE PATENTSCHRIFT

(11) **EP 2 726 216 B1**
(45) Veröffentlichungstag und Bekanntmachung des Hinweises auf die Patenterteilung: **24.10.2018**
(21) Anmeldenummer: 12731431.8
(22) Anmeldetag: 29.06.2012
(51) Int. Cl.: A61Q 15/00, A61K 8/04, B05B 7/24, B05B 11/00, B65D 83/38, B65D 83/68, A61Q 13/00

(54) **SPENDER FÜR SPRÜHBARE ZUBEREITUNGEN**
DISPENSER FOR SPRAYABLE PREPARATIONS
DISTRIBUTEUR DE PRÉPARATIONS PULVÉRISABLES

(30) Priorität: 30.06.2011 DE 102011078476
(43) Veröffentlichungstag der Anmeldung: 07.05.2014
(73) Patentinhaber: Beiersdorf AG, 20253 Hamburg (DE)
(72) Erfinder: BIEL, Stefan, 21077 Hamburg (DE); MIERTSCH, Heike, 22459 Hamburg (DE); MENZEL, Norbert, 21244 Buchholz (DE)
(74) Vertreter: Hartmann, Jost
(86) Internationale Anmeldenummer: PCT/EP2012/062661
(87) Internationale Veröffentlichungsnummer: WO 2013/001048

(56) Entgegenhaltungen:
- EP-A2- 0 092 359
- WO-A2-2006/018593
- DE-C- 917 240
- FR-E- 89 652

## Beschreibung

Die Erfindung beschreibt individualkosmetische Spender umfassend mindestens zwei Behältnisse, in denen unterschiedliche Zubereitungen vorhanden sind, wobei ein Behältnis vorteilhaft lösbar mit dem anderen Behältnis verbunden ist und über eine Zuleitung die beiden Zubereitung erst beim Ausbringen zusammengeführt werden.

Unter Individualkosmetik wird bislang den nach einer genauen Hautanalyse ermittelten individuellen Pflegebedarf der Haut und dem anschließenden Anfertigen der individuellen Kosmetikcreme verstanden.

Das Fertigen ist bislang auf das Anmischen von Zubereitungen mit individuell gewählten Bestandteilen beschränkt. Eine einmal gemischte Zubereitung, eine Hautcreme oder ein Parfum, ist dann nicht mehr individuell variierbar.

Insbesondere bei Deodorant und/oder Antitranspirant (Deo/AT) Produkten erwartet der Verbraucher neben einer Basis-Produktleistung verschiedene Vorteile, wie beispielsweise Hautpflege nach der Achselrasur, verschiedene Duftvarianten je nach Stimmung und verwendetem Parfum oder eine besondere Wirkleistung für besonders herausfordernde Situationen mithilfe spezieller Wirksysteme. Aufgrund des zumeist beschränkten Platzes im Badezimmer und weil es sich bei Deo/AT-Produkten um klassische Produkte handelt, ist der Verbraucher normalerweise allerdings nicht bereit, sich für jeden dieser Zwecke ein eigenes Deoprodukt ins Badezimmer zu stellen.

Basierend auf diesen Bedürfnissen ist es demnach wünschenswert für individualisierbare kosmetische, insbesondere Deo/AT, Produkte ein Basissystem mit verschiedenen Zusatzeigenschaften zur Verfügung zu stellen, wobei die Zusatzeigenschaften je nach Anforderungsprofil einfach und schnell integriert werden können.

EP 737629 A2 beschreibt einen Spender für schaumförmige Zubereitungen. Der Schaumstrang wird dabei durch eine Pumpeinrichtung aus einer Ausgabeöffnung freigegeben. Beim Austreten des Schaumstranges drückt dieser auf die benachbart angeordneten Behältnisse, die mit einer zusätzlichen flüssigen Zubereitung gefüllt sind. Dadurch werden die flüssigen Zubereitungen über eine Öffnung dem Schaumstrand zudosiert und gemeinsam ausgetragen. Das Ausgabeprinzip ist hierbei wie bei den streifenförmigen Zahnpastatuben zu sehen. Die Zusatzbehälter der flüssigen Zubereitungen erlauben über verschiedene farbliche Gestaltung eine Unterscheidung der unterschiedlichen Zubereitungen.

In bekannten Aerosol-Zerstäubern wird eine darin enthaltene Flüssigkeit durch ein in dem Behälter vorhandenes Treibmittel unter Druck gehalten und zur Bildung eines zumeist konischen Zerstäuberstrahls durch eine Düse ausgetrieben. In einer möglichen Ausführungsform wird dabei das flüssige Produkt von einem eine Venturidüse durchströmenden, unter Druck stehenden, gasförmigen Treibmittel aus dem Produktbehälter in die Venturidüse angesaugt. Anschließend wird das flüssige Produkt und das gasförmige Treibmittel als Sprühstrahl oder -nebel aus der Venturidüse ausgestoßen. Beispiele derartiger Zerstäuber sind in US 3672545, US 3677525 oder US 3733010 offenbart.

DE 2451367 A2 beschreibt Aerosolzerstäuber zum Abgeben eines aus einem Gemisch aus Flüssigkeit und Gas bestehenden Sprühnebels, mit wenigstens einem Behälter für ein zu zerstäubendes flüssiges Produkt und wenigstens einer innerhalb des Behälters oder getrennt von diesem angeordneten Quelle für ein Treibmittel.

US 6116466 beschreibt einen Spender zur Ausgabe zweier Zubereitungen über eine Ausgabeöffnung. Auch hier werden die Zubereitungen der parallel angeordneten Zusatzbehältnisse über einen Druck der Basiszubereitung auf die Kolben der Zusatzbehältnisse ausgegeben.

WO 2008118446 A2 beschreibt ein nachfüllbares System für Haushaltsreiniger mit einem Verdünnungsmittel in einem Vorratsbehälter und mehreren wechselbaren Behältern, die den eigentlichen Reiniger enthalten.

In den Dokumenten ist ein einfaches Auswechseln der Zusatzbehältnisse ohne Verlust an Ventil- und Strömungseinrichtungen nicht resourceschonend realisierbar.

Wünschenswert ist es daher ein Spendersystem zur Verfügung zu stellen, bei dem die Zusatzbehältnisse für die zuzumischende Zubereitung einfach und ohne Verluste von Produkt oder Performanceeinbußen am eigentlichen Spendersystem ausgewechselt werden können.

DE 60213052 D1 offenbart eine Zerstäubervorrichtung umfassend mindestens einen Produkteintritt, der in der Lage ist mit einem Vorrat dieses Produkts in Fluidverbindung gebracht zu werden, wobei letzteres in den Vorrat mit Hilfe eines Unterdrucks gesaugt wird, der in der Nähe dieses mindestens einen Produkteintritts mittels einer Abgabe dieses Trägergases erzeugt wird.

Das Trägergas wird dabei von mindestens zwei Gasaustrittsöffnungen abgegeben, deren jeweilige Lagen so gewählt sind, dass die von diesen Öffnungen abgegebenen Gasstrahlen sich treffen.

DE 60128831 D1 offenbart eine Zerstäubervorrichtung umfassend eine Reserve von zu zerstäubendem Produkt und einen ein Gas enthaltenden Behälter sowie ein Ventil, das, wenn es betätigt wird, die Zerstäubung des Produkts gestattet, wobei dieses feste Teilchen und mindestens einen Gelbildner enthält. Die Produktreserve ist dabei in einem anderen Behälter enthalten als demjenigen, der das Gas enthält, oder das Produkt ist in einem biegsamen Beutel enthalten, der im Inneren des das Gas enthaltenden Behälters angeordnet ist.

DE 1935960 A1 offenbart eine Vorrichtung zum Versprühen eines Mischproduktes, dessen Komponenten zwar bis zum Gebrauch isoliert vorliegen, jedoch zur Bildung des zu versprühenden Endproduktes müssen die Komponenten vorab gemischt werden.

EP 737629 A1 beschreibt einen Spender für schaumartige Formulierungen, denen flüssige Bestandteile zugemischt und gemeinsam ausgetragen werden ähnlich den Zahnpastastreifen.

FR 2589832 A1 offenbart 2-KammerSysteme, bei denen beide Kammern übereinander angeordnet sind. Die Zubereitungen der beiden Kammern werden in einem statischen Mischer zusammengeführt, reagieren dort miteinander und bilden einen Schaum, der dann aushärtet.

FR 89652 E beschreibt einen Spender für sprühbare Zubereitungen, der zwei Behältnisse, eine Ventileinrichtung und entsprechende Zuleitungen offenbart.

Es wird auch auf die Inkompatibilität zweier Zubereitungen hingewiesen, die gemeinsam zu applizieren sind.

DE 917240 beschreibt einen Flüssigkeitszerstäuber, bei dem die Flüssigkeit durch einen Druckgas- oder Druckluftstrom angesaugt und zerstäubt wird.

EP 92359 A2 beschreibt einen Flüssigkeitsdispenser. Auch hierin werden zwei Behältnisse mit unterschiedlichen Zubereitungen offenbart, die über Ventil- und Auslasswege zum gemeinsamen Applizieren gestaltet sind.

WO 2006018593 A2 offenbart einen Zubereitungsdispenser sowie das Prinzip eines per Folie verschlossenen und per Dorn zu öffnenden Wechselbehälters.

Die Erfindung umfasst einen Spender für sprühbare Zubereitungen entsprechend Anspruch 1.

In den Abbildungen 1 bis 7 sind die Bestandteile des erfindungsgemäßen Spenders näher erläutert.

Abbildung 1 zeigt den schematischen Aufbau einer bevorzugten Ausführungsform des Spenders.

Abbildung 2 zeigt eine Vergrößerung des Spenderkopfes mit Pump-/Ventilvorrichtung und Aufsatzbehältnis der bevorzugten Ausführungsform.

Abbildung 3 zeigt eine Vergrößerung des Zuleitungsprinzips der Ergänzungszubereitung in die Auslassöffnung des Spenders der bevorzugten Ausführungsform.

Abbildung 4 und 4a zeigen die bevorzugte Ausführungsform mit konisch auslaufender Austrittsöffnung.

Abbildung 5 zeigt drei verschiedene Auslassöffnungen mit unterschiedlich positionierten Zuläufen.

Abbildung 6 zeigt den Spenderkopf mit innenliegendem Ergänzungsbehälter.

Abbildung 7 zeigt den Spender mit innenliegendem Ergänzungsbehälter und einem Restriktor. Zur Verbesserung der Verständlichkeit wird im Weiteren für die Zubereitung (2) und (6) sowie die Behälter (1) und (5) immer die Einzahl verwendet, ohne hierdurch die Erfindung auf jeweils nur eine Zubereitung bzw. nur einen Behälter einzuschränken.

Der Spender umfasst neben den baulichen Merkmalen auch Zubereitungen in den entsprechenden Behältnissen. Auch die Zubereitungsmerkmale kennzeichnen den Spender, so dass der Spender als Kit aus Behältnissen, Vorrichtungsmerkmalen und Zubereitung besteht. Die mit dem erfindungsgemäßen Spender erzeugten Zubereitungen, bestehend aus der Zubereitung (2) aus Behälter (1) und der Ergänzungszubereitung (6) aus dem Ergänzungsbehälter (5), sind sprühbar. D.h. sie lassen sich zerstäuben, auch Sprühen genannt. "Sprühen" ist das Zerteilen einer Flüssigkeit in feinste Tröpfchen, d.h. als Aerosol oder Nebel, in einem Gas, wie z.B. Propan, Butan, Stickstoff oder Luft. Das entstehende Aerosol wird auch Spray oder Sprüh genannt. Dieses kann entweder aus Tropfen bestehen, welche alle denselben Durchmesser aufweisen - monodisperses Spray - oder verschieden große Tropfen enthalten. In diesem Fall spricht man von einem polydispersen Spray.

Hochviskose Zubereitungen wie Pasten, z.B. Zahnpasta, oder Schäume sind erfindungsgemäß nicht unter dem Begriff sprühbare Zubereitungen zu verstehen. Zum Unterschied versteht man unter einem Schaum (von lat. spuma) gasförmige Bläschen, welche von festen oder flüssigen Wänden eingeschlossen sind, also keine verteilbaren Flüssigkeitstropfen umfassen.

Die Ergänzungszubereitung (6) ist zumindest im Temperaturbereich von 0° C bis +50°C, insbesondere bei Raumtemperatur fließfähig.

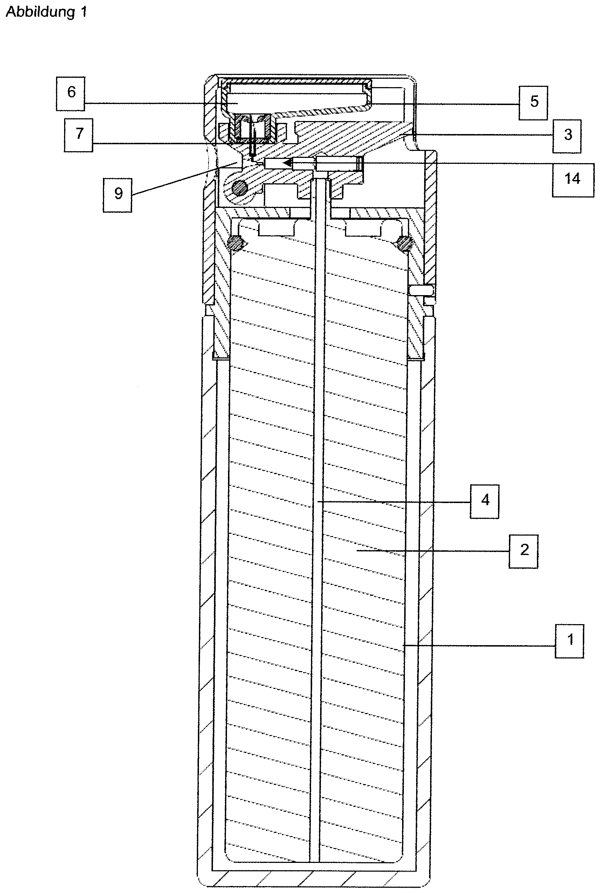

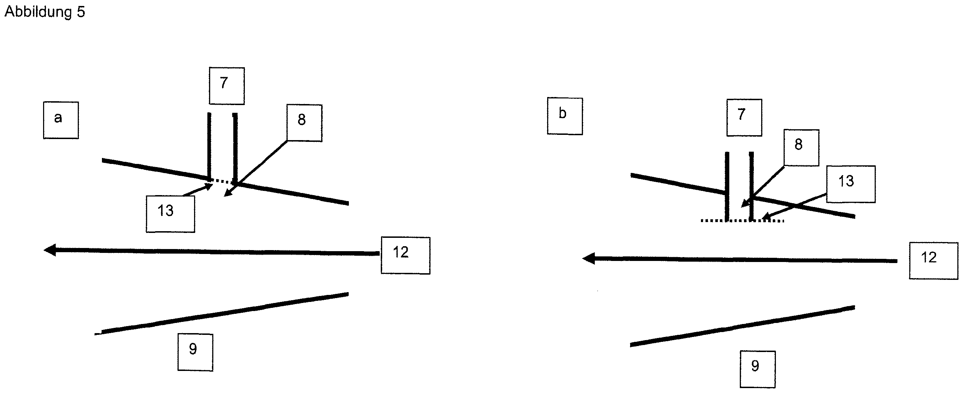

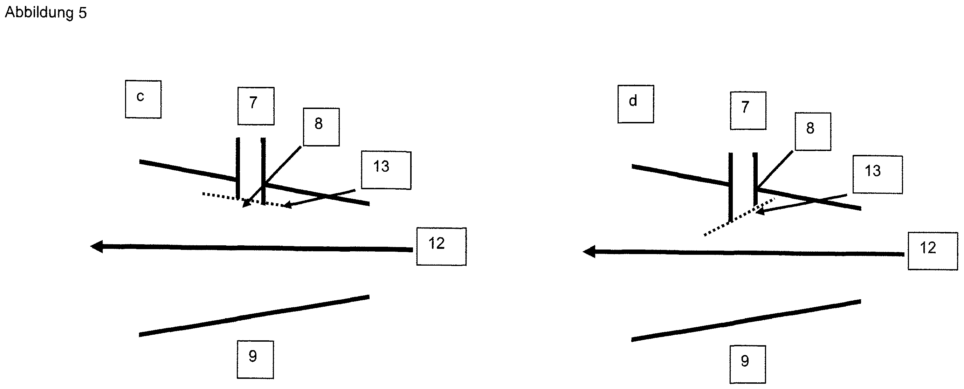

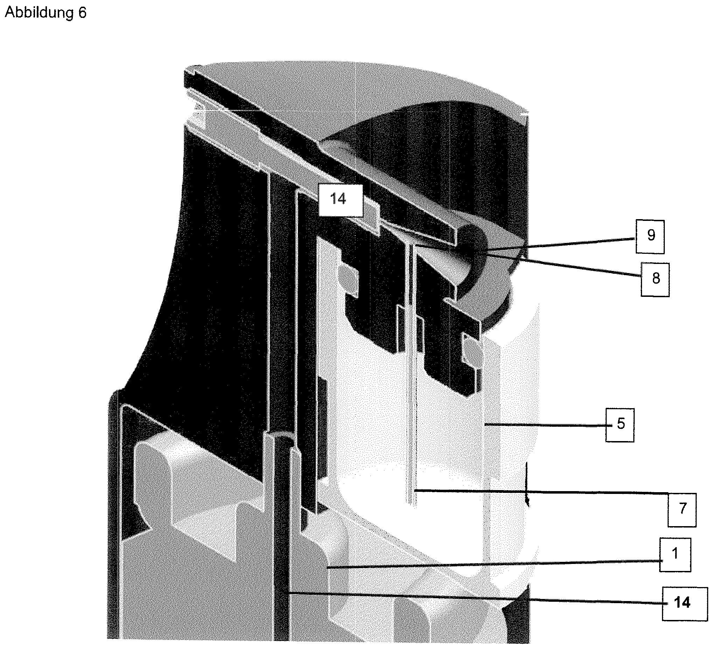

Die Auslassöffnung (9) ist im Bereich der Zuleitung(7, 8) konisch gestaltet. In Abbildungen 1 bis 7 ist dieser Konus in bevorzugten Ausführungsformen dargestellt. Der Konus ist erfindungsgemäß zum Auslass gerichtet weiter werdend gestaltet. Der Durchmesser des Konus der Auslassöffnung (9) wird zum Auslass größer, d.h. die Nennweite des kegelförmigen Bereichs nimmt in Richtung des Sprühstrahls (12), zum Auslass hin zu (Abbildungen 5).

Der Konuswinkel kann dabei vorteilhaft von 5° bis 70°, insbesondere im Bereich von 8° bis 20°, idealerweise im Bereich um 10° bis 15° gewählt werden.

Die Auslassöffnung (9) ist, wie der Name sagt, am Auslass des Spenders angebracht. Erst hier werden die Zubereitungen (2, 6) zusammengeführt. Eine vorherige Mischung, wie es in vielen Stand der Technik-Spendern offenbart wird, findet nicht statt. Bei Betätigen der Pump- oder Ventileinrichtung (3) strömt die Zubereitung (2) aus dem Behälter (1), ggf. über ein Steigrohr (4), über einen Förderkanal (14) in die Auslassöffnung (9). Im konisch gestalteten Bereich (8) reißt der Zubereitungsstrom (2, 12) die Ergänzungszubereitung (6) aus dem Ende der Zuleitung (7) mit.

Der erfindungsgemäße Spender umfasst daher auch keine extra zu diesem Zweck vorgesehene Mischeinrichtung, Mischkammer oder ähnliche Vorrichtungsmerkmale zum Mischen von Zubereitungen. Der erfindungsgenmäße Spender umfasst demnach bevorzugt keine Mischeinrichtung, die die beiden Zubereitungen (2, 6) vor der Auslassöffnung zusammenbringt.

Durch dieses erst zum Ende gestaltete Zusammenführen der Zubereitungen (2, 6) ist gewährleistet, dass auch eigentlich miteinander unverträgliche Zubereitungsbestandteile erfindungsgemäß gemeinsam ausgetragen werden können.

Die Zuleitung (7) endet in der Auslassöffnung (9) in dem Bereich, der konisch gestaltet ist (8). Die Zuleitung (7) kann dabei mit dem Konusrand abschließen (Abbildung 5a) oder in die Auslassöffnung hineinragen (Abbildungen 5b und c). Ein Hineinragen der Zuleitung (7) über die Konusmitte hinaus (Abbildung 5 - Konusmitte durch den Sprühstrahl 12 gekennzeichnet) ist zu vermeiden. Die Zuleitung (7) sollte so positioniert sein, dass ihre Öffnung (13) in einen Bereich innerhalb des Konus hineinragt, der so gewählt ist, das hier eine ausreichend hohe Strömungsgeschwindigkeit des Zubereitungsstromes herrscht um eine ausreichende Förderleistung für die Ergänzungszubereitung (6) zu gewährleisten.

Vorteilhaft ist dabei das Ende der Zuleitung (13) parallel zur Sprühstrahlrichtung (12) zu gestalten (siehe Abbildung 5b). Zu vermeiden ist, dass das Ende der Zuleitung (13) parallel zur gegenüberliegenden Konuswand gestaltet ist und Gestaltungen derart, dass das Ende der Zuleitung (13) wie es in Abbildungen 5d skizziert ist.

Das Ende der Zuleitung (7, 8) ist in Strömungsrichtung möglichst nah am Beginn des Konusbereiches, d.h. an der engsten Stelle des Konus positioniert. Teilt man die gesamte konusförmige Auslassöffnung in zwei gleiche Teile, so findet sich das Ende der Zuleitung (7, 8) in der ersten Hälfte ausgehend vom Förderkanal (14) gesehen.

Vorteilhaft ist um die Auslassöffnung (9) ein Schacht (16) ausgebildet, um Tropfen der ausgesprühten Zubereitungen (2) und (6) zu reduzieren bzw. zu verhindern.

In einer bevorzugten Ausführungsform sind der oder die Aufsatzbehälter (5) lösbar mit dem Behälter (1) und/oder der Pump/- oder Ventileinrichtung (3) verbunden.

Ein bevorzugter Aspekt ergibt sich auch aus der erfindungsgemäßen Lösbarkeit, indem der Aufsatzbehälter (5) unlösbar mit der Ventil/Pumpeinheit (3) verbunden ist und diese komplette Einheit dann wiederum lösbar vom dem Behälter (1) ist, so dass der Konsument den kompletten Kopf nach Belieben tauschen kann.

Bevorzugt ist es, dass Aufsatzbehälter (5), Pump-/Ventileinheit (3) und Auslöser (bspw. als Taster) (15) eine feste Einheit bilden, die vom Konsument als ein Teil wahrgenommen wird, und diese Einheit komplett lösbar mit dem Behälter (1) verbunden ist, so dass der Konsument die komplette Einheit nach Belieben tauschen kann.

In einer weiteren bevorzugten Ausführungsform ist der Zubereitungsbehälter (1) ebenfalls oder anstelle lösbar von der Pump- und/oder Ventileinrichtung (3) oder dem Kopf konzipiert.

In einer Vorrichtung ist dann die Verbindungseinheit für die beiden Zubereitungsbehälter fest integriert, umfassend die Ventil- und/oder Pumpeinrichtung (3), sowie die entsprechenden Zuleitungen (7). Der Behälter (1) als auch Aufsatzbehälter (5) sind von dieser Vorrichtung lösbar und somit beide Behältnisse (1, 5) individuell austauschbar.

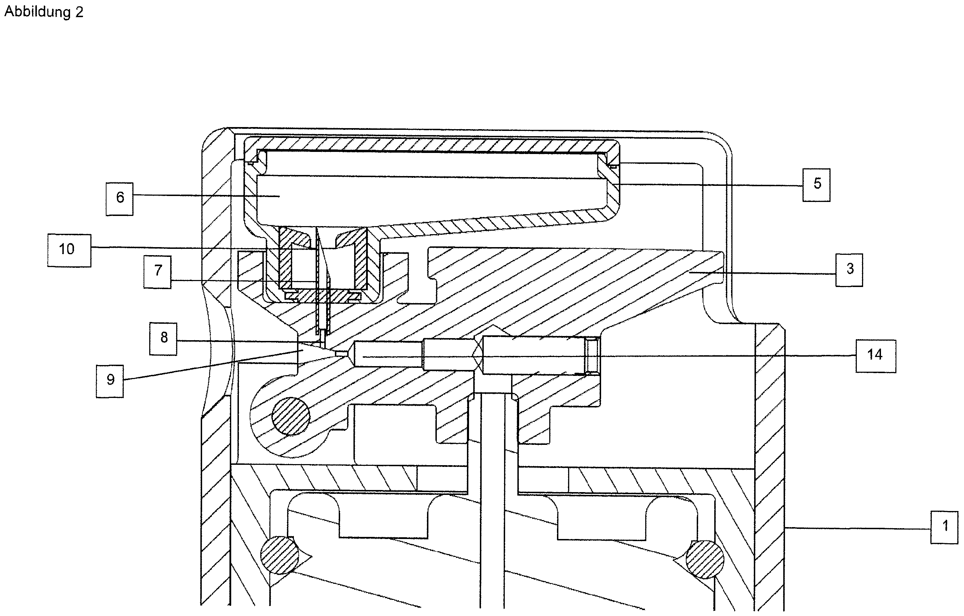

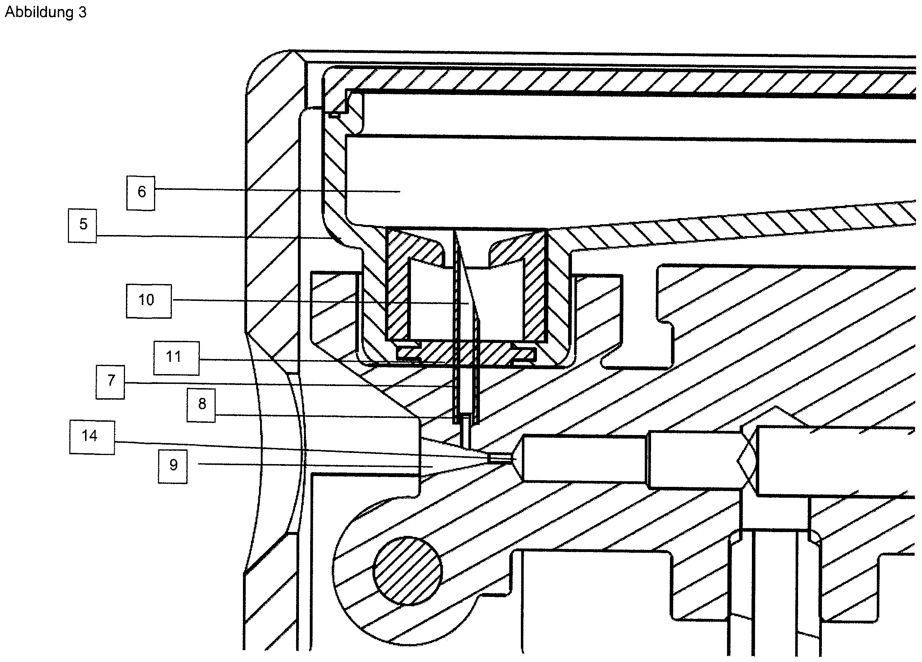

Erfindungsgemäß ist ein Ende (8) der Zuleitung (7) so angeordnet, dass es in die Auslassöffnung (9) der Pump-/Ventileinrichtung (3) ragt bzw. an der Auslassöffnung (9) mündet und das andere Ende (10) der Zuleitung (7) in den Aufsatzbehälter (5) ragt bzw. mündet. Dieser Bereich, das Herzstück des erfindungsgemäßen Systems, wird aus den Verbindungseinheiten (7, 8, 9,10, siehe Abbildungen 2 und 3) gebildet. In dieser Verbindungseinheit werden zwischen den beiden Zubereitungsbehältnissen (1, 5) die beiden Zubereitungen (2, 6) erst zusammengeführt und ausgetragen.

Wird durch Betätigen der Pumpvorrichtung (15, 3) Druck auf das Behältnis (1) ausgeübt, kann die Zubereitung (2) ggf. über das Steigrohr (4) und den Förderkanal (14) über die Auslassöffnung (9) ausgegeben werden.

Vorteilhaft ist im Behälter (1) ein Ventil eingebracht, mit dem die Pump-/Ventileinheit (3) verbunden ist. Bei Betätigung der Pump-/Ventileinheit (3), z. B. über den Auslöser (15), wird das Ventil im Behälter geöffnet und die Zubereitung (2) strömt über den Förderkanal (14) zur Auslassöffnung (9).

In einer bevorzugten Ausführungsform weist Aufsatzbehälter (5) einen für das Zuleitungsende (10) flüssigkeitsdichten Einlass (11) auf, der bevorzugt als durchstossbare Folie ausgebildet ist.

Entsprechend bevorzugt ist das Zuleitungsende (10) als Dorn gefertigt.

In einer weiteren bevorzugten Ausführungsform ist das eine Ende (8) der Zuleitung (7) in Richtung Auslassöffnung (9) in dem Aufsatzbehälter (5) integriert. Das Ende (10) der Zuleitung (7) wird als Steigrohr ausgebildet, welches in den Aufsatzbehälter ragt.

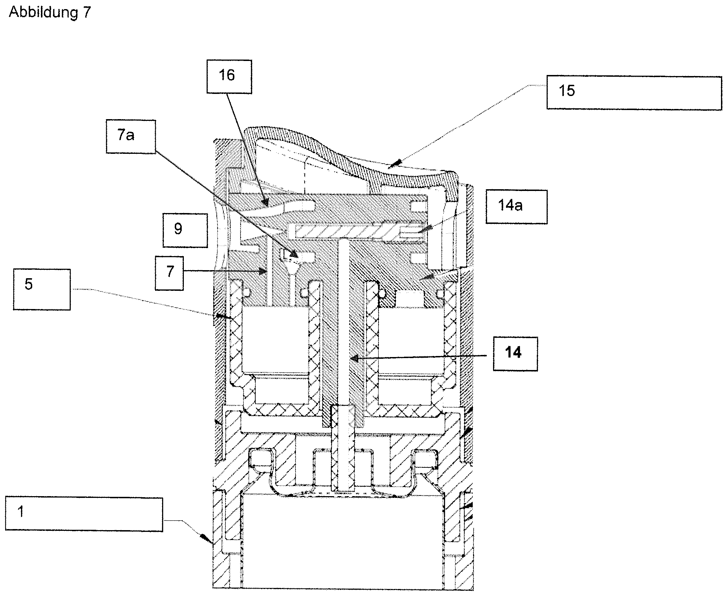

Es ist ebenfalls vorteilhaft, dass die Pump-/Ventileinheit (3) einschliesslich als Deckel des Aufsatzbehälter (5) ausgebildet ist (siehe Abbildung 7). Der Deckel beinhaltet dann die Zuleitung (7), ggf. mit Steigrohr, die Entlüftungsöffnung (7a), die Auslassöffnung (9), den Förderkanal (14) und einen zusätzlichen Schacht (16) um die Auslassöffnung.

Andere Schließ- bzw. Verbindungsvorrichtungen im Bereich des Aufsatzbehälters (5) und des Zuleitungsendes (10) sind frei wählbar.

Der Aufsatzbehälter (5) weist bevorzugt eine Belüftungsöffnung (7a) auf.

So ist ein Kupplungsmechanismus denkbar, der beim Betätigen des Ventils oder Pumpe (3) zeitgleich die Verbindung zwischen der Zuleitung (7) und dem Aufsatzbehältnis (5) sowie die Belüftungsöffnung (7a) öffnet bzw. schließt.

Die Ventileinrichtung (3) muss nicht zwingend mit dem Förderkanal (14) zusammengefügt sein. Beispielsweise gehört das Ventil (3) zum Behälter (1), die Pumpeinrichtung (3) ist dann eher eine Art Auslöser und enthält das Herzstück des System, nämlich die Verbindungseinheit (7, 8, 9, 10).

In Abbildung 7 ist der Auslöser (15) als Druckknopf am Spenderkopf vorgesehen.

Im Förderbereich kann ein Restriktor (14a) vorgesehen sein, der der Regulierung des Volumenstromes dient.

Die Aufsatzbehälter (5) können verschiedenste Ergänzungszubereitungen umfassen. Beispielsweise umfasst jeder einzelne Aufsatzbehälter eine andere Parfumzusammensetzung.

Die Basiszubereitung (2) im Behälter (1) ist dann beispielsweise eine Deodorant- und/oder Antitranspirantzubereitung jedoch ohne Parfüm.

Der Anwender kann dann je nach seinen individuellen Wünschen einen passenden Duft aus den Ergänzungszubereitungen wählen und einen entsprechenden Aufsatzbehälter auf seinen Spender aufsetzen. Nach Anwendung kann er den Aufsatzbehälter dann wieder abnehmen und bei anderer Gelegenheit einen anderen Aufsatzbehälter mit anderer Parfumzusammensetzung wählen.

Erfindungsgemäßer Vorteil dieser Ausstattungsvariante, Zubereitung (2) umfasst eine Antitranspirantzubereitung und die Ergänzungszubereitung (6) umfasst Parfüme, ist, dass eine mögliche Zersetzung von Parfumbestandteilen durch das AT-mittel, wie Aluminiumchlorohydrate, vermieden wird.

Die nachfolgende Tabelle führt vorteilhafte Zubereitungsvarianten auf.

| Zubereitung 2 | Ergänzungszubereitung 6 |
|---|---|
| reines Treibgas, z. B. Gemisch aus Propan, Butan, Isobutan | Konzentrat aus mindestens einem Parfüm, Deowirkstoff und einem Lösemittel |
| reines Treibgas, z. B. Gemisch aus Propan, Butan, Isobutan | Konzentrat aus mindestems einem Parfüm, AT-Wirker und Lösemittel |
| reines Treibgas, z. B. Gemisch aus Propan, Butan, Isobutan | Konzentrat enthaltend mindestens ein Parfum und Wirkstoffe mit hautpflegenden Eigenschaften (z.B. Q10) |
| Treibgas mit alkoholische Lösung und Deowirker | Parfum |
| Treibgas mit wässriger Lösung und Deowirker | Parfüm, Lösemittel und zusätzlich Wirkstoffe |
| Treibgas, Antitranspirantzubereitung mit ATwirkstoff | Parfum |
| Treibgas, Antitranspirantzubereitung mit ATwirkstoff | Parfum, Lösemittel und Wirkstoff |

Die Zubereitungen, gebildet aus den beiden Zubereitungen im Behältnis (1) und der Ergänzungszubereitung (6), können beliebig gewählt werden.

Vorteilhaft werden als Zubereitungen diejenigen gewählt, so dass die Zubereitung (2) mit der Ergänzungszubereitung (6) nicht homogen mischbar ist.

Ebenso bevorzugt enthält die Ergänzungszubereitung (6) ein oder mehrere Bestandteile, die nicht kompatibel mit dem Behälter (1) und/oder der Zubereitung (2) sind.

Vorteilhaft werden als Ergänzungszubereitungen (6) Parfumhaltige Zubereitungen und/oder als Zubereitung (2) parfumfreie Deodorant- und/oder Antitranspirantzubereitungen gewählt. Neben Deodorant oder Antitranspirantzubereitungen sind im erfindungsgemäßen Spender vor allem sprühbare Zubereitungen zur Pflege oder Stayling der Harre (Haarspray), sprühbare Sonnenschutzzubereitungen, Körpersprays, Zubereitungen zur Pflege der Haut und Zubereitungen zur Desinfektion von Oberflächen anwendbar.

Vorteilhafte Zubereitungskombinationen (2, 6) ergeben sich aufgrund bestehender Inkompatibilitäten der Zubereitungen oder einzelner Inhaltsstoffe. So ist es erfindungsgemäß möglich zwei Zubereitungen, die sich nicht homogen mischen lassen, getrennt in den jeweiligen Behältnissen aufzubewahren und dann dennoch gemeinsam in gewünschten Verhältnissen miteinander zu applizieren.

Der erfindungsgemäße Spender lässt sich somit zum gemeinsamen Ausbringen von ein oder mehreren Zubereitungen verwenden, deren einzelne Bestandteile sich nicht homogen mischen lassen und/oder die inkompatibel zueinander und/oder mit den Behältnissen sind. Erfindungsgemäß ist es nun möglich wässrige Aluminiumchlorohydrat haltige Zubereitungen auszutragen ohne Korrosionsprobleme metallischer Behältnisse (1) zu befürchten, indem die wässrige ACH-Zubereitung in den Ergänzungsbehältnissen (5), beispielsweise aus Plastik, enthalten ist.

Mit Hilfe der erfindungsgemäßen Vorrichtung können ein oder mehrere Einzelkomponenten einer Zubereitung durch den Anwender beliebig gewechselt werden (z.B. über die Aufsatzbehältnisse (5)). Desgleichen wird der Träger für die kosmetische Zubereitung oder die Basiszubereitung an sich beliebig oft wiederverwendet (über Behältnis (1)).

Bei Verwendung von mechanischen oder elektrischen Pumpen, einem Bag-in-Can oder Bag-on-Valve System und bei Systemen, bei denen der Druck auf andere Art und Weise erzeugt wird, entfällt ggf. das Treibgas in Zubereitung (2) bzw. liegt nicht gemischt mit der Zubereitung vor. Bei Bag-on-Valve ist die Zubereitung in einem Beutel und im umgebenden Behälter selbst nur das Treibgas. Das Behältnis (1) umfasst dann sowohl den Beutel, die darin enthaltende Zubereitung als auch das Treibgas.

Die Aufsatzbehälter (5) lassen sich beispielsweise und ohne Einschränkung auf diese Auswahl mittels Schiebearretierung, Klickmechanismus, Drehmechanismus, Klemmvorrichtung, Kupplung, magnetischer Verbindung oder ähnlichen Systemen lösbar auf dem Behälter (1) und/oder der Pump- bzw. Ventilvorrichtung (3) befestigen. Eine einfache Entnahme und Austausch der Aufsatzbehältnisse ist damit gegeben.

Wenn Aufsatzbehälter (5), Pump-/Ventileinheit (3) und Taster (15) eine feste Einheit bilden, ist diese mit den beispielhaft genannten Mechanismen wie Schiebearretierung, Klickmechanismus, Drehmechanismus, Klemmvorrichtung, Kupplung, magnetischer Verbindung oder ähnlichen Systemen lösbar auf dem Behälter (1) verbunden.

Vorteilhaft sind die Volumina des Behälters (1) und des Aufsatzbehälters (5) unterschiedlich. Dies ist für den Gebrauch als Individualkosmetik zweckmäßig, da die Basiszubereitungen nicht so häufig ausgetauscht werden müssen und daher ein größeres Volumen einnehmen sollte.

Bevorzugt ist das Verhältnis des Volumens des Behälters (1) zum Volumen des Aufsatzbehälters (5) im Bereich von 200:1 bis 1:1 zu wählen, bevorzugtes Verhältnis ist im Bereich von 100:1 bis 2:1 zu wählen.

Der Spender ist vorteilhaft so konzipiert, dass auf dem Basisbehälter (1) die notwendigen erfindungsgemäßen Vorrichtungsbestandteile (3, 7, 9) installiert sind und der Aufsatzbehälter (5) mittels zuvor erwähnter Verbindungsmechanik mit dem Behältnis verbunden wird. Die Ergänzungsbehälter ragen vorteilhaft nicht über die Abmessungen des Behältnisses (1) hinaus bzw. werden in Öffnungen, die auf dem Behälter (1) konzipiert werden, eingeschoben.

Der Anwender hat somit, wie gewohnt, eine kosmetische Aerosol- oder Pumpvorrichtung zur Hand mit den ihm bekannten Sprühvorrichtungen und kann sich die individuellen Zusatzbenefit generierenden Zubereitungen dazu kombinieren.

Entscheidender Vorteil ist, dass die Aufsatzbehältnisse lösbar und damit austauschbar gestaltet werden können. Vorteilhaft können die verschiedenen Aufsatzbehältnisse (5), die beispielsweise unterschiedliche Duftzusammensetzungen umfassen, unterschiedlich farbig gestaltet sein, um ein einfaches wiedererkennbares Auswählen ermöglicht.

Der erfindungsgemäße Spender umfasst eine Pump- und/oder Ventileinrichtung, ggf. darin integriert ein Förderkanal, um ein Sprühen bzw. Zerstäuben einer kosmetischen Zubereitung zu ermöglichen. Mit Zerstäuben bzw. Sprühen ist das Zerteilen einer Zubereitung in Tröpfchen bzw. Partikel als Aerosolnebel gemeint. Dafür eignen sich insbesondere Zubereitungen, die eine Viskosität unterhalb von 10000 mPas bei einer Schergeschwindigkeit von 10 1/s, vorzugsweise unterhalb von 5000 mPas, aufweisen. Die Viskosität zur Beurteilung des Sprühverhaltens bezieht sich hier beim Vorhandensein von Treibgasen auf die gemessene Viskosität der Zubereitung ohne Treibgas. Die Zubereitungen können darüber hinaus auch Partikel enthalten. Die beim Sprühen entstehenden Tröpfchen bilden in der Regel ein polydisperses Spray, d.h. die Tröpfchen sind verschieden groß. Idealerweise ist der Anteil an lungengängigen Tröpfchen / Partikeln, damit sind Tröpfchen / Partikel kleiner 10µm gemeint, möglichst gering.

Die Pumpvorrichtung (3) ist vorteilhaft als mechanisches Pumpsystem ausgeführt, wie es aus den bekannten Pumpzerstäubern bekannt ist. Ebenso sind aber auch elektrische Pumpvorrichtungen (3) zur Druckerzeugung im Behälter (1) möglich.

Die erfindungsgemäßen Zubereitungen werden bevorzugt dünnflüssig und sprühbar formuliert, damit sie u.a. vorteilhaft als Aerosol applizierbar sind.

Die Zubereitung (6) in den Aufsatzbehältern (5) ist vorteilhaft bei Raumtemperatur, insbesondere im Temperaturbereich von 0°C bis + 40°C und Normaldruck flüssig.

Die Zubereitung (2) im Basisbehälter (1) ist bevorzugt eine unter Druck stehende Aerosolzubereitung.

Ein Aerosol ist ein disperses System, bei dem ein Feststoff oder eine Flüssigkeit in einem Gas äußerst fein verteilt vorliegt. Das Aerosol wird in der Regel erst bei der Anwendung mit Hilfe eines geeigneten Sprühsystems durch Versprühen von Lösungen, Emulsionen oder Suspensionen selbst erzeugt, wozu beispielsweise Sprühdosen verwendet werden können, in denen ein verflüssigtes Druckgas als Treibgas dient. Beim Öffnen des Druckventils (3) entweicht das Treibmittel-Zubereitungsgemisch (2) durch eine feine Düse (9), das Treibmittel verdampft und hinterlässt das fein verteilte Sprühgut als Aerosol. Im anwendungstechnischen Sprachgebrauch wird der Begriff Aerosol vom Fachmann häufig auch im Sinne von Aerosolsprays verwendet, d.h. hier wird unter "Aerosol" nicht der reine Sprühnebel, sondern eine Druckgasverpackung nebst Abgabe- bzw. Dosiervorrichtung und Füllgut verstanden.

Als Treibmittel sind die üblichen "klassischen" leichtflüchtigen, verflüssigten Treibgase, wie beispielsweise Dimethylether (DME) und/oder lineare oder verzweigtkettige Kohlenwasserstoffe mit zwei bis fünf Kohlenstoffatomen (wie insbesondere Ethan, Propan, Butan, Isobutan und/oder Pentan, Isopentan) geeignet, die allein oder in Mischung miteinander eingesetzt werden können.

Auch Druckluft sowie weitere unter Druck befindliche Gase wie Luft, Sauerstoff, Stickstoff, Wasserstoff, Helium, Krypton, Xenon, Radon, Argon, Lachgas (N₂O) und Kohlendioxid (CO₂) sind vorteilhaft im Sinne der vorliegenden Erfindung als Treibgase (sowohl einzeln als in beliebigen Mischungen miteinander) zu verwenden.

Natürlich weiß der Fachmann, dass es weitere an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere halogenierte (mit Fluor, Clor, Brom, lod und/oder Astat substituierte) Kohlenwasserstoffe wie beispielsweise Fluorkohlenwasserstoffe und Fluorchlorkohlenwasserstoffe (FCKW).

Die genannten Gase können im Sinne der vorliegenden Erfindung jeweils einzeln oder in beliebigen Mischungen zueinander verwendet werden. Besonders vorteilhafte Sprays sind erhältlich, wenn die erfindungsgemäßen Zubereitungen mit Hilfe von linearen oder verzweigtkettigen Kohlenwasserstoffen, insbesondere Mischungen aus Propan, Butan und Isobutan, versprüht werden.

Vorteilhaft liegt das Verhältnis der Innendrucke des Behälters (1) zu dem Innendruck des Ergänzungsbehälters (5) im Bereich von 50:1 bis 1:1.

Der Innendruck des Ergänzungsbehälters (5) ist idealerweise gleich dem Umgebungsdruck (1 bar).

Abweichungen sind erfindungsgemäß möglich, zumal über einen leichten Über- bzw. Unterdruck das Verhalten der Zubereitungssysteme bei der Ausbringung oder auch hinsichtlich der Tropfgröße verändert werden kann.

Strömungstechnik und -mechanik der erfindungsgemäßen Systeme sind von den zugrundeliegenden Durchmessern bzw. Verhältnissen der Auslassöffnung (9) sowie von den verwendeten Treibgasen und Druckstufen abhängig. Die Sprührate ist hauptsächlich vom Ventil und dem Öffnungsdurchmesser für die Zuleitung (7) sowie von den rheologischen Eigenschaften der versprühten Zubereitungen abhängig. Die Sprührate, die ausgetragende Menge an Zubereitung pro Zeiteinheit, hängt somit von der Zusammensetzung der Zubereitung und den Strömungsverhältnissen ab. Dabei wird die Sprührate der Zubereitung vor allem über das verwendete Ventil bei einem treibgasbasiserten System bzw. über die Pumpengeometrie bei einem treibgasfreien System sowie die Geometrien in der Verbindungseinheit (3, 9,14) bestimmt. Die Sprührate der Ergänzungszubereitung (6) ergibt sich hauptsächlich über die Ausführung der Zuleitung (7), insbesondere den Durchmesser der Zuleitung und der Querschnitte im Unterdruckbereich (8) und an der Auslassöffnung (9). So erhöht sich beispielsweise die Sprührate der Ergänzungszubereitung je größer der Durchmesser der Zuleitung (7) gewählt wird.

Bevorzugt werden die Innendurchmesser der Zuleitungen (7) im Bereich von 0,3 bis 1,5 mm, bevorzugt 0,5 bis 0,7 mm, gewählt.

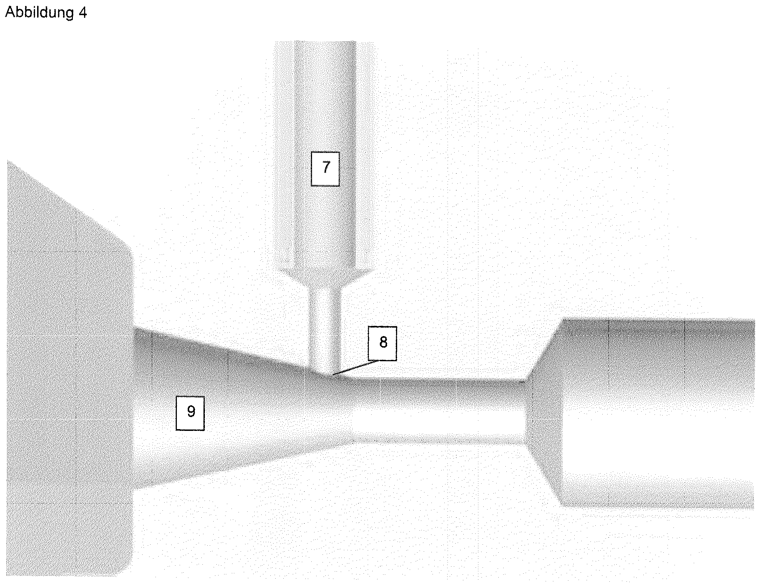

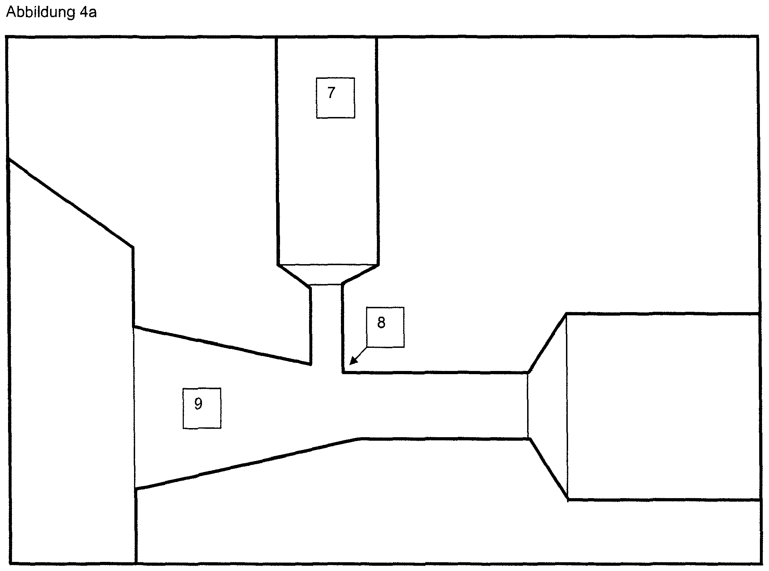

Die Geometrie der Auslassöffnung (9) im Bereich (8) wird konisch ausgeführt mit einem nach außen weiter werdenden Öffnungsbereich, wie es insbesondere in Abbildung 4 dargestellt ist. Zum Ende des konischen Zuführbereiches (8, 9) kann die Auslassöffnung beliebig gestaltet sein, wobei eine Verringerung der Nennweite, also eine Durchmesserreduzierung, zu vermeiden ist.

Die erfindungsgemäße Auslassöffnung ist daher keine Düse mit zum Auslass geringer werdendem Querschnitt.

Der erfindungsgemäße Spender erzeugt ein gegenüber Standardaerosol und Pumpzerstäubern allein aufgrund der konischen Form der Auslassöffnung ein besseres Sprühbild. D.h. gleichmäßigere Tröpfchenverteilung, weniger Ausreißer, zielgerichteter Sprühstrahl.

Vorteil des erfindungsgemäßen Spenders ist, dass bei Ausbringung gleicher Wirkstoffmengen der Anteil an Treibgas verringert ist gegenüber Zubereitungen und Spendern des Standes der Technik. Das Besondere ist, dass erfindungsgemäß der Behälter der unter Druck steht gegenüber einem herkömmlichen Aerosol verkleinert werden kann ohne Einbußen hinsichtlich der ausgebrachten Wirkstoffmenge. Somit wird das Volumen der als Gefahrgut geltenden Produktteile verringert, was wiederum umweltfreundlicher und kostengünstiger ist.

Herkömmliches Aerosol umfassend eine Dose mit 150 ml gilt als Gefahrgut.

Durch die Aufteilung in getrennte Behältnisse kann die als Gefahrgut zu deklarierenden Behältnisse verkleinert werden (jetzt nur noch 75 ml).

Die dann beispielsweise umfassende 25 ml Ergänzungszubereitung gilt nicht als Gefahrgut.

Es ergibt sich eine grundsätzliche Einsparung in der Menge (150 ml vs. 100 ml) als auch in der besonderen Logistik (150 ml gefahrgut zu 75 ml).

Vorteilhaft umfasst der Spender ein oder mehrere Deodorant- und/oder Antitranspirantzubereitungen, die sich aus dem Zusammenfügen der Zubereitungen 2 und 6 bei der Applikation ergeben.

Die Aufteilung, welcher Zubereitungsteil als Zubereitung 2 oder 6, oder welche konkreten Inhaltsstoffe dieser Zubereitungen in den Basisbehälter (1) oder die Aufsatzbehälter (5) gegeben werden sind individuell variierbar.

Kosmetische Antitranspirantien oder Desodorantien/Deodorantien dienen dazu, Körpergeruch zu beseitigen bzw. deren Entstehung zu vermindern. Körpergeruch entsteht, wenn der an sich geruchlose frische Schweiß durch Mikroorganismen wie z.B. Staphylokokken und Corynebakterien zersetzt wird.

Den üblichen kosmetischen Desodorantien liegen unterschiedliche Wirkprinzipien zugrunde.

Im allgemeinen Sprachgebrauch erfolgt nicht immer ein klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die - im Gegensatz zu den Desodorantien, die im Allgemeinen eine mikrobielle Zersetzung von bereits gebildetem Schweiß verhindern - die Absonderung von Schweiß überhaupt verhindern sollen.

Die gewünschte Minimierung der Schweißsekretion kann durch verschiedene Mechanismen realisiert werden. Hierzu zählt traditionell der Einsatz von Adstringenzien, welche Eiweißfällungen und Gerinnungen hervorrufen und so eine Verengung oder Verschluss der Schweißdrüse bewirken.

Neuartige AT-Wirker basieren bspw. auf dem Prinzip der Anticholinergika, welche die Nervenreize, die zur Sekretionssteigerung der Schweißdrüsen führen, unterbrechen. Ein weiteres Prinzip neuartiger AT-Wirker beruht auf der Beeinflussung von Membrantransportvorgängen in der Zelle. So hemmen spezifische Aquaporin-Inhibitoren die Proteine, die Kanäle in der Zellmembran bilden, um den Durchtritt von Wasser und weiteren Molekülen zu erleichtern. Auch lonenkanal-Hemmer bewirken eine Beeinflussung von Membrantransport- bzw. Osmosevorgängen. Ein weiterer neuartiger AT-Wirkmechanismus lässt sich durch die Verwendung kurzkettiger, vicinaler Diole realisieren, welcher möglicherweise auf deren osmotischen Aktivität zurückzuführen ist.

So durch Anwendung eines Mittels kein Einfluss auf die Schweißsekretion ausgeübt wird, also keine antitranspirante Wirkung realisiert wird, liegt erfindungsgemäß kein Antitranspirant vor.

Erfindungsgemäß sind demnach als Antitranspirantwirkstoffe solche Stoffe umfasst, die einen Einfluss auf die Schweißsekretion haben.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruchs (Geruchsverbesserungsmittel). Gängige Wirkmechanismen hierzu sind antibakterielle Effekte, wie sie z.B. auch das nicht-kolloidale Silber zeigt, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Reaktionen zu wohlriechenden Stoffen umgesetzt werden.

Zubereitungen können neben den eigentlichen schweißhemmenden Wirkstoffen (AT-Wirker) zusätzlich auch Stoffe enthalten, die den mikrobiellen Abbau des Schweißes hemmen, wie z. B. Butyloctanoic Acid. Butyloctanoic Acid zeigt jedoch nur eine desodorierende Wirkung und keine antitranspirante Wirkung, da aus der Beeinflussung der bakteriellen Hautflora keine Beeinflussung der Schweißsekretion abzuleiten ist.

Insbesondere bei Antitranspirantien auf Basis von Aluminiumsalzen kann ggf. auf den weiteren Zusatz von rein desodorierend wirkenden Substanzen verzichtet werden, da diese per se ein antimikrobielles Potential aufweisen. Des Weiteren wird durch das reduzierte Wasserangebot durch die verminderte Schweißsekretion auch das bakterielle Wachstum gehemmt.

Antitranspirantwirkstoffe im Sinne der vorliegenden Anmeldung sind insbesondere aus den folgenden Gruppen zu wählen.

Als Antitranspirantwirkstoffe finden insbesondere Adstringenzien Verwendung, vornehmlich Aluminiumverbindungen. Die früher eingesetzten, stark sauer wirkenden Salze Aluminiumsulfat oder -chlorid und die Agaricinsäure sind weitgehend durch Aluminiumhydroxychlorid und -alkoholate ersetzt worden. Die nachfolgende Auflistung vorteilhaft einzusetzender Antitranspirant-Wirker soll in keinster Weise einschränkend sein: Aluminiumsalze:
- Aluminiumsalze wie Aluminiumchlorid AlCl3, Aluminiumsulfat Al2(SO4)3
- Aluminiumchloride der empirischen Summenformel [Al2(OH)mCln], wobei m+n=6
- Aluminiumchlorhydrat [Al2(OH)5Cl] x H2O
   ▪ Standard Al-Komplexe: Locron P (Clariant), Micro-Dry 323 (Summit Reheis), Aloxicoll PF 40 (BK Giulini).
   ∘ Aktivierte Al-Komplexe: Reach 103 (Summit Reheis), AACH-7171/AACH-7172 (Summit Reheis), Aloxicoll P (BK Giulini), Aloxicoll SD100 (BK Giulini)
- Aluminiumsesquichlorhydrat [Al2(OH)4,5Cl1,5] x H2O Standard Al-Komplexe: Aloxicoll 31P (BK Giulini)
- Aluminiumdichlorhydrat [Al2(OH)4Cl2] x H2O

Die Aluminiumsalze können in gemahlener Form oder auch als Hohlkugeln oder als Mischung dieser vorliegen. Die Dichte dieser Partikel liegt vorteilhaft im Bereich von 0,7 bis 2,0 g/cm³.

Die kosmetischen oder dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Polyole, Polymere, Schaumstabilisatoren, organische Lösungsmittel oder Silikonderivate, sofern der Zusatz die geforderten Eigenschaften hinsichtlich der Ausbrinbarkeit aus dem Spender nicht beeinträchtigt.

Die nachfolgenden Beispiele zeigen nur einen geringen Ausschnitt an Möglichkeiten, welche der erfingungsgemäße Spender bietet. Die Zahlenangaben stellen, sofern nichts anderes angegeben, Gewichtsanteile dar, bezogen auf die Gesamtmasse der jeweiligen Zubereitung.

### Variante I:

Im Behälter (1) ist nur Treibgas enthalten. Der austauschbare Aufsatzbehälter (5) kann verschiedenste komplette Zubereitungen enthalten. Die Konzentration der einzelnen Bestandteile der Ergänzungszubereitung hängt von der Ausführung des Systems ab.

### Zubereitung 2

| | Beispiel 21 | Beispiel 22 | Beispiel 23 |
|---|---|---|---|
| Treibgas Druckstufe 2.7 bar | 100% | | |
| Treibgas Druckstufe 2.1 bar | | 100% | |
| Treibgas Druckstufe 1.1 bar | | | 100% |

### Ergänzungszubereitung 6

| Beispiel 61 | Beispiel 62 | Beispiel 63 | Beispiel 64 |
|---|---|---|---|
| Parfüm | Parfüm | Parfüm | Lösemittel |
| Lösemittel, z. B. Ethanol | Lösemittel | Lösemittel | Deowirker |
| Deowirker | AT-Wirker | Deowirker | Hautbefeuchtungsmittel |
| | | AT Wirker | |

### Variante II

Im Behälter (1) ist eine Zubereitung mit Deowirkern enthalten, die bereits ohne die Ergänzungszubereitung im Aufsatzbehälter (5) wirksam ist. Die Ergänzungszubereitung ermöglicht die Auswahl des Parfüms und weiterer Pflege- und/oder Wirkstoffe. Die Konzentrationsangaben in den Zubereitungen gelten für einen Spender, bei dem die in der Abbildung dargestellten Geometrien maßstabsgerecht umgesetzt wurden.

### Zubereitung 2

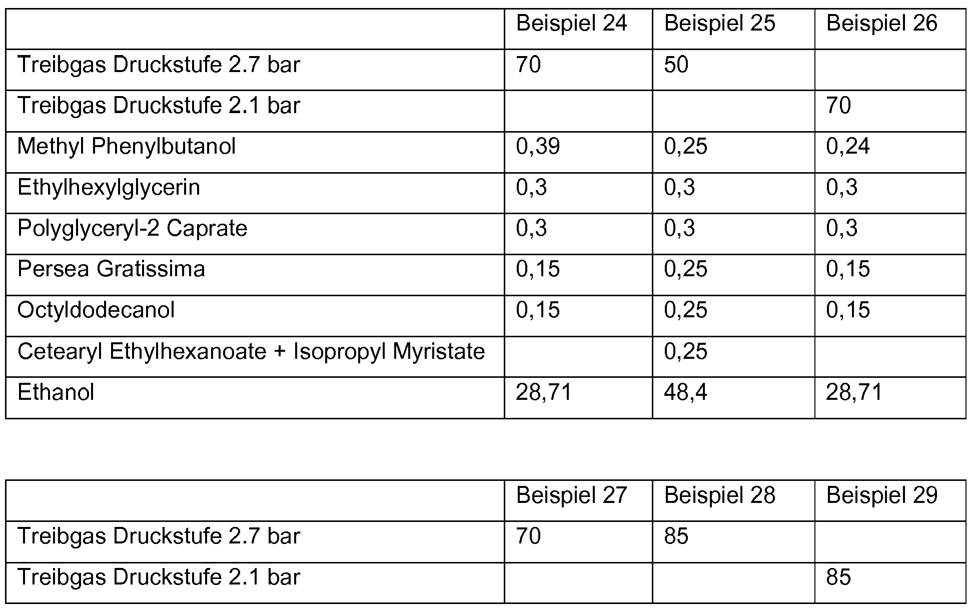

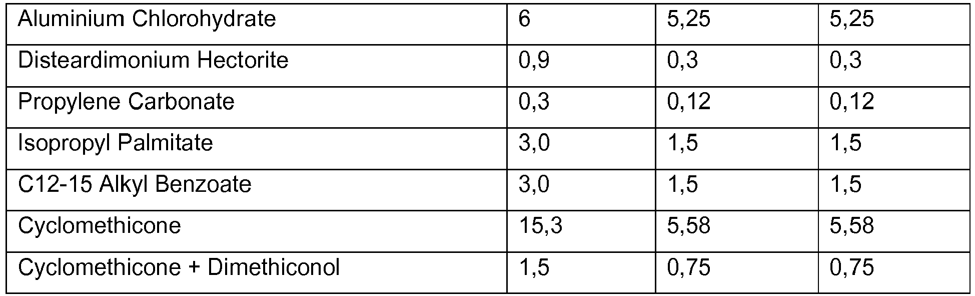

### Ergänzungszubereitung 6

Die Ergänzungsbereitung besteht nur aus Parfüm.

### Variante III

### Zubereitung 2

| | Beispiel 210 | Beispiel 211 | Beispiel 212 |
|---|---|---|---|
| Treibgas Druckstufe 2.7 bar | 70 | 85 | |
| Treibgas Druckstufe 2.1 bar | | | 85 |
| Aluminium Chlorohydrate | 6 | 5,25 | 5,25 |
| Disteardimonium Hectorite | 0,9 | 0,3 | 0,3 |
| Propylene Carbonate | 0,3 | 0,12 | 0,12 |
| Isopropyl Palmitate | 3,0 | 1,5 | 1,5 |
| C12-15 Alkyl Benzoate | 3,0 | 1,5 | 1,5 |
| Cyclomethicone | 15,3 | 5,58 | 5,58 |
| Cyclomethicone + Dimethiconol | 1,5 | 0,75 | 0,75 |

Die Ergänzungsbereitung besteht nur aus Parfüm.

Als Ergänzungszubereitungen (6) werden demnach bevorzugt parfumhaltige Zubereitungen gewählt und als Zubereitung (2) parfumfreie Deodorant- und/oder Antitranspirantzubereitungen gewählt werden.

## Patentansprüche

1. Spender für sprühbare Zubereitungen umfassend
a. mindestens einen Behälter (1) umfassend mindestens eine Zubereitung (2), eine Pump- und/oder Ventileinrichtung (3), eine Auslassöffnung (9), einen Förderkanal (14), ggf. ein Steigrohr (4) für die Zubereitung (2) und mindestens eine Zuleitung (7), und
b. ein oder mehrere Ergänzungsbehälter (5) umfassend ein oder mehrere fließfähige Ergänzungszubereitungen (6),
**dadurch gekennzeichnet, dass**
ein Ende (8) der Zuleitung (7) in die Auslassöffnung (9) und das andere Ende (10) der Zuleitung (7) in den oder die Ergänzungsbehälter (5) mündet,
die Auslassöffnung (9) zumindest im Bereich (8) der Zuleitung (7) konisch gestaltet ist,-der Durchmesser des Konus zum Auslass hin größer wird,
die Zuleitung (7) dabei mit dem Konusrand abschließt oder in die Auslassöffnung (9) hineinragt, wobei das Hineinragen der Zuleitung (7) über die Konusmitte hinaus (12) vermieden wird, und
das Ende der Zuleitung (7, 8) in der ersten Hälfte ausgehend vom Förderkanal (14) des Konusbereiches angeordnet ist.

2. Spender nach Anspruch 1 **dadurch gekennzeichnet, dass** der oder die Ergänzungsbehälter (5) lösbar mit dem Behälter (1) verbunden sind.

3. Spender nach Anspruch 1 **dadurch gekennzeichnet, dass** der oder die Ergänzungsbehälter (5) mit der Pump/- oder Ventileinrichtung (3) und einem Auslöser (15) verbunden sind und zusammen lösbar mit dem Behälter (1) verbunden sind.

4. Spender nach einem der vorstehenden Ansprüche **dadurch gekennzeichnet, dass** der Konus einen Winkelbereich von 8 bis 20°, insbesondere im Bereich um 10 bis 15° aufweist

5. Spender nach Anspruch 1, **dadurch gekennzeichnet, dass** der Ergänzungsbehälter (5) einen für das Zuleitungsende (10) flüssigkeitsdichten Einlass (11) aufweist.

6. Spender nach Anspruch 5, **dadurch gekennzeichnet, dass** der flüssigkeitsdichte Einlass (11) als durchstossbare Folie ausgebildet ist.

7. Spender nach Anspruch 6, **dadurch gekennzeichnet, dass** das Zuleitungsende (10) als Dorn gefertigt ist.

8. Spender nach einem der vorstehenden Ansprüche, **dadurch gekennzeichnet, dass** die Volumina des Behälters (1) und des Ergänzungsbehälters (5) ein Verhältnis im Bereich von 200:1 bis 1:1, insbesondere 100:1 bis 2:1 , aufweisen.

9. Spender nach einem der vorstehenden Ansprüche, **dadurch gekennzeichnet, dass** eine mechanische oder elektrische Pumpvorrichtung (3) zur Druckerzeugung im Behälter (1) vorhanden ist.

10. Spender nach Anspruch 9, **dadurch gekennzeichnet, dass** der Innendruck in den Behältern (1) und Ergänzungsbehältern (5) ein Verhältnis im Bereich von 50:1 bis 1:1 aufweist.

11. Spender nach einem der vorstehenden Ansprüche, **dadurch gekennzeichnet, dass** die Innendurchmesser der Zuleitungen (7) im Bereich von 0,3 bis 1,5 mm, bevorzugt 0,5 bis 0,7 mm, gewählt werden.

12. Spender nach einem der vorstehenden Ansprüche, **dadurch gekennzeichnet, dass** die Zubereitung (6) bei Raumtemperatur flüssig ist.

13. Spender nach einem der vorstehenden Ansprüche, **dadurch gekennzeichnet, dass** die Zubereitung (2) mit der Ergänzungszubereitung (6) nicht homogen mischbar ist.

14. Spender nach einem der vorstehenden Ansprüche, **dadurch gekennzeichnet, dass** die Ergänzungszubereitung (6) ein oder mehrere Bestandteile enthält, die nicht kompatibel mit dem Behälter (1) und/oder der Zubereitung (2) sind.

15. Spender nach einem der vorstehenden Ansprüche, **dadurch gekennzeichnet, dass** als Ergänzungszubereitungen (6) parfumhaltige Zubereitungen gewählt werden.

16. Spender nach einem der vorstehenden Ansprüche, **dadurch gekennzeichnet, dass** als Zubereitung (2) parfumfreie Deodorant- und/oder Antitranspirantzubereitungen gewählt werden.

17. Spender nach einem der vorstehenden Ansprüche umfassend keine Mischeinrichtung, die die beiden Zubereitungen (2, 6) vor der Auslassöffnung zusammenbringt.

18. Verwendung eines Spenders nach einem der vorstehenden Ansprüche zum gemeinsamen Ausbringen von ein oder mehreren Zubereitungen, deren einzelne Bestandteile sich nicht homogen mischen lassen und/oder die inkompatibel zueinander und/oder mit dem oder den Behältnissen sind.

## Claims

1. Dispenser for sprayable preparations, comprising
a. at least one container (1) comprising at least one preparation (2), a pump and/or valve device (3), an outlet opening (9), a delivery channel (14), optionally a dip tube (4) for the preparation (2), and at least one feed line (7), and
b. one or more supplementary containers (5) comprising one or more free-flowing supplementary preparations (6),
**characterized in that**
one end (8) of the feed line (7) opens into the outlet opening (9) and the other end (10) of the feed line (7) opens into the one or more supplementary containers (5),
the outlet opening (9) has a conical shape, at least in the region (8) of the feed line (7),
the diameter of the cone increases towards the outlet,
the feed line (7) ends flush with the cone edge or protrudes into the outlet opening (9), wherein protrusion of the feed line (7) beyond the cone centre (12) is avoided, and
the end of the feed line (7, 8) is arranged in the first half starting from the delivery channel (14) of the cone region.

2. Dispenser according to Claim 1, **characterized in that** the one or more supplementary containers (5) are connected releasably to the container (1).

3. Dispenser according to Claim 1, **characterized in that** the one or more supplementary containers (5) are connected to the pump and/or valve device (3) and to a trigger (15) and are together connected releasably to the container (1).

4. Dispenser according to one of the preceding claims, **characterized in that** the cone has an angle range of 8 to 20°, particularly in the range of 10 to 15°.

5. Dispenser according to Claim 1, **characterized in that** the supplementary container (5) has a liquid-tight inlet (11) for the feed line end (10).

6. Dispenser according to Claim 5, **characterized in that** the liquid-tight inlet (11) is configured as a pierceable foil.

7. Dispenser according to Claim 6, **characterized in that** the feed line end (10) is produced as a spike.

8. Dispenser according to one of the preceding claims, **characterized in that** the volumes of the container (1) and of the supplementary container (5) have a ratio in the range from 200:1 to 1:1, in particular 100:1 to 2:1.

9. Dispenser according to one of the preceding claims, **characterized in that** a mechanical or electrical pump device (3) is present for generating pressure in the container (1).

10. Dispenser according to Claim 9, **characterized in that** the internal pressure in the containers (1) and supplementary containers (5) has a ratio in the range from 50:1 to 1:1.

11. Dispenser according to one of the preceding claims, **characterized in that** the internal diameters of the feed lines (7) are chosen in the range from 0.3 to 1.5 mm, preferably 0.5 to 0.7 mm.

12. Dispenser according to one of the preceding claims, **characterized in that** the preparation (6) is liquid at room temperature.

13. Dispenser according to one of the preceding claims, **characterized in that** the preparation (2) is not homogeneously miscible with the supplementary preparation (6).

14. Dispenser according to one of the preceding claims, **characterized in that** the supplementary preparation (6) contains one or more constituents that are not compatible with the container (1) and/or the preparation (2).

15. Dispenser according to one of the preceding claims, **characterized in that** perfume-containing preparations are chosen as supplementary preparations (6).

16. Dispenser according to one of the preceding claims, **characterized in that** perfume-free deodorant and/or antiperspirant preparations are chosen as preparation (2).

17. Dispenser according to one of the preceding claims, comprising no mixing device that brings the two preparations (2, 6) together before the outlet opening.

18. Use of a dispenser according to one of the preceding claims for jointly dispensing one or more preparations whose individual constituents cannot mix homogeneously and/or which are incompatible with each other and/or with the one or more containers.

## Revendications

1. Distributeur de préparations vaporisables, comprenant
a. au moins un réservoir (1) comprenant au moins une préparation (2), un système de pompage et/ou de valve (3), un orifice de sortie (9), un canal de convoyage (14), le cas échéant, un tube ascendant (4) pour la préparation (2) et au moins un conduit d'arrivée (7), et
b. un ou plusieurs réservoir(s) complémentaire(s) (5) comprenant une ou plusieurs préparation(s) complémentaire(s) (6) fluide(s), **caractérisé**
**en ce qu'**une extrémité (8) du conduit d'arrivée (7) débouche dans l'orifice de sortie (9) et l'autre extrémité (10) du conduit d'arrivée (7) débouche dans le ou les réservoir(s) complémentaire(s) (5),
**en ce qu'**au moins dans la région (8) du conduit d'arrivée (7), l'orifice de sortie (9) est conçu sous forme conique,
**en ce que** le diamètre s'agrandit en direction de la sortie,
**en ce qu'**à cet effet, le conduit d'arrivée (7) se termine sur le bord du cône ou saillit à l'intérieur de l'orifice de sortie (9), étant évité que le conduit d'arrivée (7) saillisse au-delà (12) du centre du cône et
**en ce que** l'extrémité du conduit d'arrivée (7, 8) est placée dans la première moitié, partant du canal de convoyage (14) de la région du cône.

2. Distributeur selon la revendication 1, **caractérisé en ce que** le ou les réservoir(s) complémentaire(s) (5) sont assemblés de manière amovible avec le réservoir (1).

3. Distributeur selon la revendication 1, **caractérisé en ce que** le ou les réservoir(s) complémentaires (5) sont assemblés avec le système de pompage et/ou de valve (3) et avec un déclencheur (15) et sont assemblés avec le réservoir (1) en étant amovibles en commun.

4. Distributeur selon l'une quelconque des revendications précédentes **caractérisé en ce que** le cône présente une zone angulaire de 8 à 20°, de préférence, de l'ordre de 10 à 15°.

5. Distributeur selon la revendication 1, **caractérisé en ce que** le réservoir complémentaire (5) comporte une entrée (11) étanche aux liquides pour l'extrémité du conduit d'arrivée (10).

6. Distributeur selon la revendication 5, **caractérisé en ce que** l'entrée (11) étanche aux liquides est conçue sous la forme d'un film apte à être transpercé.

7. Distributeur selon la revendication 6, **caractérisé en ce que** l'extrémité du conduit d'arrivée (10) est fabriquée sous forme d'épine.

8. Distributeur selon l'une quelconque des revendications précédentes, **caractérisé en ce que** les volumes du réservoir (1) et du réservoir complémentaires (5) présentent un rapport de l'ordre de 200 :1 à 1 :1, notamment de 100 :1 à 2 :1.

9. Distributeur selon l'une quelconque des revendications précédentes, **caractérisé en ce qu'**un dispositif de pompage mécanique ou électrique (3) est présent pour générer une pression dans le réservoir (1).

10. Distributeur selon la revendication 9, **caractérisé en ce que** la pression intérieure dans les réservoirs (1) et réservoirs complémentaires (5) présente un rapport de l'ordre de 50 :1 à 1 :1.

11. Distributeur selon l'une quelconque des revendications précédentes, **caractérisé en ce que** les diamètres intérieurs des conduits d'arrivée (7) sont choisis dans l'ordre de 0,3 à 1,5 mm, de préférence de 0,5 à 0,7 mm.

12. Distributeur selon l'une quelconque des revendications précédentes, **caractérisé en ce que** la préparation (6) est liquide à température ambiante.

13. Distributeur selon l'une quelconque des revendications précédentes, **caractérisé en ce que** la préparation (2) n'est pas miscible de manière homogène avec la préparation complémentaire (6).

14. Distributeur selon l'une quelconque des revendications précédentes, **caractérisé en ce que** la préparation complémentaire (6) contient un ou plusieurs composant(s), qui ne sont pas compatibles avec le réservoir (1) et/ou la préparation (2).

15. Distributeur selon l'une quelconque des revendications précédentes, **caractérisé en ce qu'**en tant que préparations complémentaires (6), sont choisies des préparations contenant du parfum.

16. Distributeur selon l'une quelconque des revendications précédentes, **caractérisé en ce qu'**en tant que préparation (2) sont choisies des préparations déodorantes et/ou anti-transpirantes sans parfum.

17. Distributeur selon l'une quelconque des revendications précédentes ne comprenant aucun système mélangeur qui réunit les deux préparations (2, 6) à l'avant de l'orifice de sortie.

18. Utilisation d'un distributeur selon l'une quelconque des revendications précédentes, pour expulser une ou plusieurs préparations dont les composants individuels ne sont pas miscibles de manière homogène et/ou qui sont incompatibles réciproquement et/ou avec le ou les réservoirs.
